(19) Europäisches Patentamt
European Patent Office
Office européen des brevets

(11) EP 0 843 652 B1

(12) **FASCICULE DE BREVET EUROPEEN**

(45) Date de publication et mention de la délivrance du brevet:
**03.11.1999 Bulletin 1999/44**

(21) Numéro de dépôt: **96928484.3**

(22) Date de dépôt: **06.08.1996**

(51) Int Cl.[6]: **C02F 3/34**

(86) Numéro de dépôt international:
**PCT/FR96/01250**

(87) Numéro de publication internationale:
**WO 97/06107 (20.02.1997 Gazette 1997/09)**

(54) **PROCEDE DE BIODEPOLLUTION D'EFFLUENTS A FORTES CONCENTRATIONS EN POLLUANTS, ET PROCEDE DE SELECTION DE SOUCHES UTILISABLE DANS LEDIT PROCEDE**

VERFAHREN ZUR BIOLOGISCHEN REINIGUNG VON EINEM HOHEN GEHALT AN SCHADSTOFFEN AUFWEISENDEN ABWÄSSERN, UND VERFAHREN FÜR DIE SELEKTION VON MIKROORGANISMENSTRÄNGEN GEEIGNET FÜR DIESES VERFAHREN

METHOD FOR BIODEPOLLUTING EFFLUENTS WITH HIGH CONCENTRATIONS OF POLLUTANTS, AND STRAIN SELECTION PROCESS USABLE WITH SAID METHOD

(84) Etats contractants désignés:
**AT BE CH DE DK ES FI FR GB GR IE IT LI LU MC NL PT SE**

(30) Priorité: **07.08.1995 FR 9509563**

(43) Date de publication de la demande:
**27.05.1998 Bulletin 1998/22**

(73) Titulaires:
- **COMMISSARIAT A L'ENERGIE ATOMIQUE**
  **75015 Paris (FR)**
  Etats contractants désignés:
  **BE CH DE ES GB IT LI SE**
- **COMURHEX**
  **Société pour la Conversion de l'Uranium en Métal et Hexafluorure**
  **78140 Velizy Villacoublay (FR)**
  Etats contractants désignés:
  **AT BE CH DE DK ES FI FR GB GR IE IT LI LU MC NL PT SE**

(72) Inventeurs:
- **PACCARD, Emmanuelle**
  **F-69210 Saint Bel (FR)**
- **BESNAINOU, Bernard**
  **F-13100 Aix-en-Provence (FR)**
- **MOLETTA, René**
  **F-11120 Le Somail (FR)**

(74) Mandataire: **Des Termes, Monique et al**
**Société Brevatome**
**3, rue du Docteur Lanceraux**
**75008 Paris (FR)**

(56) Documents cités:
**EP-A- 0 562 466**
**EP-A- 0 634 368**
**WO-A-91/01278**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a trait à un procédé de biodépollution (ou encore d'épuration biologique) d'effluents renfermant de fortes concentrations en polluants organiques ou en éléments minéraux susceptibles d'être réduits ou oxydés par des bactéries, en particulier l'invention a trait à un procédé de dénitrification biologique d'effluents à forte concentration en nitrates qui consiste à mettre en oeuvre une population mixte de bactéries dénitrifiantes. L'invention a également trait à un procédé de sélection de souches performantes pour la biodépollution d'effluents très chargés en polluants qui est basé sur l'utilisation de ces mêmes effluents comme base pour la préparation des milieux de culture. Ce procédé de sélection est en particulier utilisable pour sélectionner une population mixte de bactéries dénitrifiantes à hautes performances à partir de prélèvements réalisés sur le site industriel-même générant les effluents à dénitrifier. L'invention concerne également la souche dominante de cette population mixte de bactéries dénitrifiantes, cette souche étant une souche nouvelle qui a été déposée auprès de la Collection Nationale de cultures de Microorganismes sous le numéro CNCM I 1563.

Il existe de nombreux polluants organique et minéraux présentant des effets nocifs, en particulier, les effets nocifs des nitrates sont bien connus ; ils consistent notamment en une toxicité vis-à-vis de la vie aquatique, en la production d'algues dans les cours d'eau et les lacs, en un appauvrissement de l'eau en oxygène dissous, en une réduction de l'efficacité de la désinfection par le chlore, en une réduction de la capacité de réutilisation des effluents, et en une toxicité aiguë et chronique pour l'homme, en particulier pour le nourrisson à des concentrations au-dessus de 10 mg/litre. Le rejet d'effluents chargés en nitrate est donc devenu de moins en moins souhaitable et se heurte à des réglementations restrictives.

La technique de biodénitrification ou de dénitrification biologique se définit comme la réduction des ions nitrates ou nitrites en azote moléculaire ou en oxydes d'azote sous l'action d'une bactérie. Cette technique a été appliquée avec succès à la dénitrification des effluents urbains, des effluents issus de l'agriculture ou encore des eaux de consommation peu chargées en- nitrate : c'est-à-dire présentant une concentration généralement inférieure à 1 g/l.

Pour les concentrations plus élevées qui sont celles rencontrées dans les effluents industriels, de nombreux procédés non-biologiques ont tout d'abord été proposés : il s'agit par exemple d'une neutralisation par la chaux suivie soit d'une calcination, soit d'une transformation en engrais liquide ou solide, soit d'un enfouissement. Tous ces procédés présentent l'inconvénient de nécessiter entre autres l'utilisation de quantités importantes de réactifs, et de fortes consommations d'énergie.

Les procédés de dénitrification biologique peuvent également être appliqués aux effluents industriels fortement chargés, mais ils ont l'inconvénient de présenter une vitesse de réaction lente et de nécessiter une dilution importante des effluents à traiter. D'autre part, les effluents industriels chargés en nitrates tels que les effluents issus des installations nucléaires, contiennent fréquemment des métaux lourds et d'autres inhibiteurs potentiels de la dénitrification biologique qui rendent difficile leur mise en oeuvre.

Cependant, de nombreuses tentatives, dont aucune ne s'est avérée entièrement satisfaisante, ont été réalisées pour dénitrifier des effluents à fortes teneurs en nitrates et notamment des effluents issus de l'industrie nucléaire.

Ainsi, C.W. Francis et al dans le document CONF 800447 (1980) décrivent-ils des essais de traitement de déchets riches en nitrate issus de l'industrie nucléaire dans lesquels sont testés trois types de réacteurs différents, dont un réacteur continu agité, alimenté par des effluents synthétiques reconstitués composés de nitrate d'ammonium, de calcium et de sodium.

La culture dénitrifiante utilisée est une culture issue d'un sédiment marin dont les capacités de dénitrification se sont avérées supérieures à celles de cultures issues d'un réacteur de dénitrification ou d'un sol agricole.

J.M. Napier et al dans Y-DA-6370 "Preprint for the Third ERDA Environmental Protection Conference, Chicago, Illinois, Septembre 23-25, 1975" étudient la dénitrification des effluents chargés en nitrates provenant d'une installation de purification de l'uranium. Trois types de réacteurs sont de nouveau testés dont un réacteur continu agité et la bactérie dénitrifiante mise en oeuvre initialement est *Pseudomonas Stutzeri.*

F.E. Clark et al dans Y-DA-6106 "Preprint for the 30 th. Annual Purdue Industrial Waste Conference, Purdue University, West Lafayette, Indiana, May 6-8, 1975" décrivent de même des tests réalisés sur des effluents issus d'une installation de purification de l'uranium par extraction au solvant et mettant en jeu trois types de réacteurs dont le plus efficace s'est avéré être le réacteur continu agité. Les réacteurs sont ensemencés avec des cultures pures de *Pseudomonas denitrificans* et de *Pseudomonas stutzeri,* puis avec des cultures mixtes.

M.E. HOLLAND et al dans GAT-2002 "Biodenitrification of gaseous diffusion plant aqueous wastes : stirred bed reactor", Goodyear atomic corporation, Piketon, Ohio, publié le 3.10.1980 décrivent la biodénitrification d'effluents aqueux provenant d'une installation de diffusion gazeuse dans un réacteur à lit agité. Le taux de dénitrification est très faible à cause des effets toxiques des métaux lourds contenus dans les effluents sur les bactéries dénitrifiantes qui ne sont pas clairement identifiées et dont la nature exacte n'est pas connue.

W.N. WHINNERY dans KY-712 "Denitrification of nitrate-containing heavy-metal-filtrate solutions" Paducah gaseous diffusion plant, publié le 1er Juillet 1981 indique que la dénitrification en continu de déchets nitratés riches

en métaux lourds issus d'une installation de diffusion gazeuse n'a pas donné de résultats satisfaisants. Les bactéries utilisées sont soit *Pseudomonas stutzeri,* soit une souche de collection, soit une bactérie provenant d'un autre réacteur de dénitrification.

G. CLAUS et al dans "Autotrophic denitrification by *Thiobacillus denitrificans* in a packed bed reactor" décrivent l'utilisation d'un réacteur à garnissage à écoulement ascendant pour la dénitrification d'effluents industriels par une culture enrichie de la bactérie *Thiobacillus denitrificans.*

On a donc cherché à sélectionner de nouvelles souches dénitrifiantes présentant des capacités de dénitrification supérieures. Ainsi DENARIAZ et al dans Int. J. of Syst. Bacteriol., pp. 145-151, 1989 décrivent-ils la nouvelle souche *Bacillus halodenitrificans* ATCC 49067 isolée à partir d'un marais salant : cette bactérie se développe dans un milieu salin et tolère de fortes concentrations en sodium, en nitrites et en nitrates. L'isolat présente toutefois l'inconvénient de ne pas posséder d'activité de réductase vis-à-vis de l'oxyde nitreux.

Cette bactérie peut donc être utilisée pour le traitement d'effluents industriels avec de fortes teneurs en nitrate sous forme de nitrate de sodium, mais du fait qu'elle n'a pas de capacité de réduction du $N_2O$, elle nécessite la mise en oeuvre d'une seconde étape de traitement.

ROBINSON et al dans Can. Journal of Botany 30 : 147-154, 1951 décrivent la souche dénommée *Micrococcus halodenitrificans* qui se développe spécifiquement dans des milieux contenant du chlorure de sodium à une forte concentration supérieure à 2,2 % et ne se développe pas en présence d'autres sels. Cette bactérie réduit les nitrates et les nitrites en azote.

TOMLINSON et al dans Int. Journal of Syst. Bac. 36, 66-70, 1986 font mention d'une nouvelle souche *Halobacterium denitrificans* qui croît exclusivement en présence de nitrates ou de nitrites et à des concentrations élevées en NaCl, supérieures à 1,5 M.

Ces souches mentionnées ci-dessus se sont avérées dans l'ensemble incapables de dénitrifier des effluents industriels à fortes concentrations en nitrates ou bien leur mise en oeuvre s'est avérée restreinte à quelques cas particuliers.

Les principaux problèmes rencontrés sont essentiellement dus soit à une sensibilité importante des souches à l'un des intermédiaires de la dénitrification, en particulier les nitrites, soit à des exigences trop restrictives en ce qui concerne la force ionique du milieu de culture, soit à l'absence chez la souche de la dernière enzyme de la chaîne de dénitrification et arrêt de la réaction au $N_2O$.

D'autres auteurs, enfin, mettent en oeuvre des flores complexes sous la forme de boues activées, non contrôlées et non caractérisées, qui très souvent présentent des capacités de dénitrification en réacteur difficilement prévisibles et maîtrisables.

Il existe donc un besoin non satisfait pour un procédé de biodépollution d'effluents à fortes concentrations en polluants organiques et/ou en éléments minéraux susceptibles d'être réduits ou oxydés par des bactéries.

Il existe en particulier un besoin jusqu'alors non satisfait pour un procédé de biodénitrification ou dénitrification bactériologique d'effluents en particulier industriels fortement chargés en nitrates. Il existe aussi un besoin pour des souches dépolluantes qui puissent être mises en oeuvre dans des effluents fortement chargés en polluants. En particulier, il existe un besoin pour des souches dénitrifiantes réalisant l'ensemble de la chaîne de dénitrification qui puissent être mises en oeuvre dans des effluents même fortement chargés en nitrates, à force ionique élevée, pouvant contenir également d'autres sels à des concentrations élevées ainsi que des éléments tels que des métaux lourds ou autres. Il y a en particulier un besoin pour un procédé permettant de sélectionner des micro-organismes qui puissent être mis en oeuvre dans un procédé de dénitrification.

L'objet de l'invention est donc de fournir un procédé de biodépollution d'effluents à fortes concentrations en polluants organiques et/ou en éléments minéraux susceptibles d'être oxydés ou réduits par des bactéries qui remplisse l'ensemble des exigences indiquées ci-dessus.

L'objet de l'invention est en particulier de fournir un procédé de dénitrification biologique remplissant l'ensemble des exigences mentionnées ci-dessus. L'invention a également pour objet un procédé de sélection de souches bactériennes et en particulier des bactéries dénitrifiantes utilisables notamment dans ledit procédé de traitement d'effluents.

Les objectifs sont atteints conformément à l'invention par un procédé de dénitrification biologique d'effluents renfermant de fortes concentrations en nitrates, par des bactéries, caractérisé en ce que la population mixte de bactéries dénitrifiantes mise en oeuvre est sélectionée par repiquages successifs à partir de prélèvements des effluents à traiter et cultivée dans des milieux de culture comprenant les effluents à traiter eux-mêmes.

Ces objectifs sont en particulier atteints conformément à l'invention par un procédé de dénitrification biologique d'effluents industriels à forte concentration en nitrates qui consiste à mettre en oeuvre une population mixte de bactéries dénitrifiantes, ces bactéries ayant été sélectionnées et cultivées dans des conditions anaérobies en utilisant les mêmes effluents comme base pour la préparation des milieux de culture. Les milieux de culture utilisés sont donc des milieux non synthétiques, non reconstitués, comme c'est le cas dans l'art antérieur.

Le procédé selon l'invention, qui est applicable en particulier à des effluents industriels, permet de réduire

les nitrates présents dans les effluents à des concentrations pouvant atteindre jusqu'à 62 g/l (1M) et ceci à une vitesse de dégradation élevée, sans accumulation temporaire ou permanente de nitrites tout en minimisant les concentrations en substrat carboné nécessaires pour la dénitrification.

Selon l'invention, on met en oeuvre dans le procédé de dénitrification une population mixte qui est sélectionnée directement à partir de prélèvements par exemple de prélèvements de sols et sédiments de sites industriels, en particulier de prélèvements dans la lagune de stockage des effluents à traiter et dont la concentration est typiquement supérieure à 100 g/l.

La sélection consiste en un enrichissement des prélèvements en bactéries dénitrifiantes dans les conditions de dénitrification et dans les effluents à traiter eux-mêmes. L'originalité de ce procédé de sélection réside d'une part dans l'origine des prélèvements destinés à servir d'"inoculum" pour les cultures de sélection : ceux-ci sont réalisés directement sur le site industriel générant des effluents nitrates et si possible directement dans les bassins ou lagunes de stockage de ces effluents, et d'autre part dans le fait que tous les milieux de cultures utilisés pour cette sélection sont composés par les effluents à traiter eux-mêmes.

Les prélèvements effectués sur le site où sont générés les effluents, sont soumis à un enrichissement par repiquages successifs. La concentration des inocula étant supérieure ou égale à 10 % v/v en milieux liquides, dans les conditions de sélection - conditions de dénitrification - imposées par les effluents à traiter. Les milieux de culture et de sélection employés sont du type de celui décrit ci-dessus : ils comportent tout d'abord des effluents à traiter qui contiennent les nitrates notamment sous forme de $KNO_3$, $NaNO_3$, $NH_4NO_3$, et $Ca(NO_3)_2$ dilués de manière à obtenir différentes concentrations en nitrates compatibles avec le problème à résoudre, par exemple les concentrations pourront varier de 5 g/l à la concentration en nitrates des effluents non dilués, par exemple de 50 g/l. Ces milieux de sélection comprennent également un substrat carboné ou source de carbone assimilable en concentration telle que les demandes en carbone pour la dénitrification et la croissance cellulaire soient satisfaites ; il peut s'agir notamment d'un acide organique ou d'un alcool tel que l'acide acétique, le méthanol, l'éthanol, ou encore d'effluents industriels chargés en polluants carbonés tels que les vinasses, les cidrasses, les effluents de l'industrie de l'amidon, les mélasses, le lactosérum ou du mélange de plusieurs de ces substrats carbonés. Les quantités de substrat carboné nécessaires à la dénitrification totale sont fonction de la quantité de nitrates à éliminer, du nombre d'électrons libérés par la réaction d'oxydation du substrat carboné selon l'équation :

$$\text{substrat carboné} + H_2O \rightarrow CO_2 + H^+ + e^-,$$

et de la production de biomasse correspondant à la réaction de dénitrification.

Par exemple, dans le cas de la souche *Pseudomonas halodenitrificans* sur acide acétique, l'oxydation totale de l'acide acétique en $CO_2$ donne $8e^-$ par mole d'acide acétique et pour la réaction de réduction des nitrates, un rapport minimal de 0,62 mole d'acide acétique/mole $NO_3^-$ est nécessaire. A cette quantité, il faut ajouter l'acide acétique consommé pour la production de biomasse, et la maintenance. Expérimentalement, dans le cas de Pseudomonas halodenitrificans sur acide acétique, un rapport molaire compris entre 0,63 et 1 de préférence supérieur à 0,8 est nécessaire pour obtenir une dénitrification totale.

Les milieux de culture et de sélection contiennent également du phosphore généralement ajouté sous la forme de phosphates : une quantité minimale de 16 mg de phosphore/g de biomasse est nécessaire et peut être apportée par exemple sous forme de $KH_2PO_4$ $Na_2HPO_4$, ou de leur mélange.

Enfin, les milieux de culture contiennent des oligo-éléments qui comprennent essentiellement Mo, Fe, S, Cu, Mg, Mn.... et leurs mélanges : la détermination d'une quantité minimale nécessaire de chacun de ces éléments n'étant pas aisée et fortement dépendante des souches mises en oeuvres, chacun d'eux sera ajouté en excès dans le milieu de culture sous la forme d'une solution d'oligo-éléments classiquement utilisée en laboratoire.

Enfin, si cela est nécessaire, une source d'azote complémentaire telle que $NH^+_4$ sous forme $NH_4Cl$ par exemple est ajoutée au milieu de culture à une concentration comprise entre 1 et 10 g/l.

La population mixte, enrichie comme décrit ci-dessus et présentant la meilleure activité de dénitrification, cette activité ayant été déterminée de la manière suivante : dosage des nitrates, dosage de la source de carbone, dosage des gaz produits, subit ensuite une deuxième étape d'enrichissement en réacteur continu agité ou autre, alimenté avec un milieu de culture de même composition que celle mentionnée ci-dessus. Dans le cas d'effluents contenant du calcium, celui-ci sera préalablement éliminé par précipitation par exemple au $K_2HPO_4$, afin d'éviter une précipitation de sels de calcium dans le réacteur pouvant inhiber la réaction de dénitrification.

Le principe de cette deuxième étape d'enrichissement consiste à effectuer un enrichissement de plus en plus poussé de la population mixte en bactéries dénitrifiantes en augmentant le taux de dilution appliqué au réacteur, par exemple jusqu'à une valeur de 0,8 $j^{-1}$.

Les souches dénitrifiantes composant la population mixte sont ensuite isolées, l'isolement étant réalisé sur une milieu solide de même composition + agar 15 g/l, et de même force ionique que le milieu d'enrichissement en

réacteur continu agité. Les boîtes de Pétri ensemencées à partir d'un aliquot de la culture en réacteur continu agité sont incubées sous hotte anaérobie à une température comprise entre 10°C et 45°C, de préférence de 20 à 40°C de préférence encore à 30°C.

L'une des souches isolées, dominante dans la culture mixte, s'est révélée, après analyses phénotypique et génétique, être une souche nouvelle, non encore décrite dans la littérature, dénommée *Pseudomonas halodenitrificans* et déposée auprès de la Collection Nationale de Cultures de Micro-organismes sous le n° CNCM I 1563.

L'invention comprend également tous les mutants, obtenus par des procédés connus de l'homme du métier, de cette souche nouvelle.

On comprendra que cette technique de sélection de souches performantes pour la biodépollution d'effluents, basée sur l'utilisation de ces mêmes effluents comme base pour la préparation des milieux de culture peut être utilisée pour sélectionner n'importe quel type de bactéries, pour la biodépollution des effluents chargés en polluants organiques ou en éléments minéraux susceptibles d'être oxydés ou réduits par ces bactéries et est donc d'application générale.

La mise en oeuvre de la culture mixte présentant la meilleure activité de dénitrification dans un procédé de dénitrification conformément à l'invention est réalisée de la manière suivante : la population mixte sélectionnée présentant la meilleure activité de dénitrification est mise en culture avec une concentration suffisante en inoculum qui sera généralement supérieure ou égale à 10 % en v/v dans un réacteur agité de production de biomasse, alimenté avec des milieux de culture composés par les effluents à traiter dilués de manière à obtenir une concentration en nitrates adéquate, généralement inférieure à 62 g/l pour un pH légèrement acide et une force ionique correspondante, une source de carbone, c'est-à-dire d'énergie exogène, éventuellement une source d'azote complémentaire autre que les nitrates par exemple du chlorure d'ammonium à une concentration $NH^+_4$ comprise entre 1g/l et 10g/l, ainsi que du phosphore, des oligo-éléments qui sont des cofacteurs des enzymes de la dénitrification.

Les nitrates sont généralement présents sous forme de $KNO_3$, et $Ca(NO_3)_2$, mais peuvent également être présents sous la forme de $HNO_3$, $NaNO_3$, ....

Des effluents fortement chargés en nitrates qui peuvent être traités par le procédé de dénitrification selon l'invention, sont générés dans de nombreux procédés industriels, en particulier dans la fabrication des engrais, dans la fabrication des explosifs, dans la fabrication de l'acide nitrique, ainsi que dans des procédés mis en oeuvre dans l'industrie nucléaire, tels que les procédés d'extraction et d'enrichissement de l'uranium, les procédés de dissolution nitrique de combustible nucléaire etc.

Des rejets d'effluents et liquides divers, riches en nitrates et qui sont susceptibles d'être traités par le procédé de l'invention peuvent également se rencontrer dans d'autres domaines notamment l'agriculture ou bien encore l'élevage etc.

La source de carbone est choisie parmi les sources carbonées connues de l'homme du métier. Dans ce domaine de la technique, par exemple on pourra utiliser un acide organique tel que l'acide acétique ou un alcool tel que le méthanol ou l'éthanol ou bien encore des effluents industriels chargés en polluants carbonés tels que les vinasses ou cidrasses, effluents de l'industrie de l'amidon, mélasses, lactosérum, ou le mélange de plusieurs de ces sources de carbone.

La source de carbone, ou substrat carboné, est présente en une concentration telle que les demandes en carbone pour la dénitrification et la croissance cellulaire soient satisfaites. Les quantités de substrat carboné nécessaires à la dénitrification totale sont fonction de la quantité de nitrates à éliminer, du nombre d'électrons libérés par la réaction d'oxydation du substrat carboné selon l'équation : substrat carboné + $H_2O \rightarrow CO_2 + H^+ + e^-$ et enfin de la production de biomasse correspondant à la réaction de dénitrification. Par exemple, dans le cas de la souche *Pseudomonas halodenitrificans* sur acide acétique, l'oxydation totale de l'acide acétique en $CO_2$ donne 8 $e^-$ par mole d'acide acétique et pour la réaction de réduction des nitrates, un rapport minimal de 0,62 mole d'acide acétique/mole $NO^-_3$ est préféré. A cette quantité, il faut ajouter l'acide acétique consommé pour la production de biomasse et la maintenance.

Expérimentalement, dans le cas de *Pseudomonas halodenitrificans* sur acide acétique, un rapport molaire minimal de 0,8 est donc préféré pour obtenir une dénitrification totale.

Le phosphore ajouté est généralement présent sous forme de phosphates : une quantité minimale de 16 mg de phosphore/g de biomasse est nécessaire et peut être apportée par exemple sous forme de $KH_2PO_4$ ou de $Na_2HPO_4$ ou de leur mélange.

Les oligo-éléments comprennent essentiellement Mo, Fe, S, Cu, Mg, Mn et leurs mélanges : la détermination d'une quantité minimale nécessaire de chacun de ces éléments n'étant pas aisée et fortement dépendante des souches mises en oeuvre, chacun d'eux sera ajouté en excès dans le milieu de culture sous la forme d'une solution d'oligo-éléments classiquement utilisée en laboratoire.

On obtient ainsi une biomasse qui peut servir à ensemencer tout type de réacteur de dénitrification contenant les effluents à traiter et fonctionnant en mode discontinu ou alimenté par ces effluents et fonctionnant alors en mode continu. On applique dans chaque cas un taux de dilution qui est adapté au type de réacteur mis en oeuvre. Ainsi, dans le cas d'un réacteur de type "batch" ou discontinu le taux de dilution est-il nul.

Pour ce qui concerne les réacteurs alimentés fonctionnant en continu parmi lesquels on peut citer les réacteurs

continus agités, les réacteurs à membranes, les réacteurs à lit fixe, les réacteurs à lit fluidisé, les réacteurs continus agités étant préférés.

Le taux de dilution appliqué est fonction de la concentration en biomasse initiale qui peut varier entre de larges limites, mais qui peut être généralement choisie entre quelques centaines de mg/l et plusieurs g/l ; le taux de dilution dépend également de la productivité spécifique des cellules et de la concentration finale en biomasse recherchée comme cela sera illustré dans les exemples 6 à 9 joints.

Il est d'autre part nécessaire de fournir des ions $H^+$, car, comme le montre l'équation de dénitrification, qui peut s'écrire sous la forme très générale suivante :

$$\text{Source de carbone et d'énergie} + NO_3^- + H^+ \rightarrow C_5H_7O_2N + CO_2 + N_2 + H_2O$$

($C_5H_7O_2N$ désignant la biomasse formée), la réaction de dénitrification consomme des ions $H^+$, c'est-à-dire que pour travailler dans des conditions de pH maîtrisées, il faut maintenir le pH à une valeur fixe, c'est-à-dire le réguler par addition d'ions $H^+$.

Les ions $H^+$ peuvent être fournis par trois voies différentes : tout d'abord par l'intermédiaire du $CO_2$ produit par la réaction, qui se répartit, en fonction de la consigne de pH fixée, entre la phase gazeuse et la phase liquide selon l'équilibre :

$$CO_{2\,gaz} \xleftrightarrow[\text{Loi de Henry}]{} CO_2\ \text{dissous} + H_2O \xleftrightarrow[\text{pk = 6,33}]{} HCO_3^- + H^+$$

Ce type de régulation du pH nécessite cependant de maintenir dans le réacteur des pressions partielles de $CO_2$ qui peuvent devenir inhibitrices pour de fortes concentrations en nitrates.

Les bicarbonates $HCO_3^-$ étant également toxiques vis-à-vis des bactéries dénitrifiantes, la réaction de dénitrification doit être conduite de manière à fournir les ions $H^+$ nécessaires à la régulation du pH sans atteindre le seuil d'inhibition par les bicarbonates. Ce seuil dépend de la population dénitrifiante mise en oeuvre. Il se situe par exemple entre 0,17 et 0,22 mol/l pour la population mixte sélectionnée ici. Ce seuil pourra être facilement déterminé par l'homme du métier en fonction de la population dénitrifiante utilisée.

La régulation du pH peut être réalisée par l'ajout d'un acide exogène. Cet acide est en général un acide minéral fort, l'acide préféré étant l'acide sulfurique concentré ou l'acide chlorhydrique.

Dans ce cas, les produits de la réaction seront du gaz carbonique $CO_2$ qui se trouvera sous la forme d'un mélange $CO_2$ gazeux, bicarbonates, carbonates, dont les proportions dépendent du pH ; des ions tels que des ions sulfates $SO_4^{2-}$ ou des ions Cl-qui proviennent de l'acide ajouté pour régulation du pH ; de l'azote gazeux ; de la biomasse ; et de l'eau.

Les nitrates présents dans les effluents et transformés en azote moléculaire au cours de la réaction sont remplacés, selon le principe de respect de l'électro-neutralité du milieu de culture, d'une part par des sulfates ou des chlorures et d'autre part par des bicarbonates produits par solubilisation du $CO_2$ généré par la réaction. Ainsi, la force ionique du milieu de culture (FI=

$$\frac{1}{2}\sum_i$$

$C_i z_i^2$ où zi représente la valence de l'ion considéré, et Ci la concentration de l'ion i), variera-t'elle en fonction des proportions en sulfates ou chlorures et bicarbonates dans le milieu de culture.

Dans le cas d'une régulation du pH de ce type, la réaction de dénitrification doit être conduite de manière à ce que les proportions en sulfates ou chlorures et bicarbonates soient telles qu'il n'y ait pas d'inhibition de la réaction de dénitrification, due soit directement à la concentration de l'une des deux espèces ioniques, soit indirectement à la force ionique du milieu de culture.

Enfin, on peut réguler le pH par l'ajout simultané par exemple d'acide sulfurique concentré ou d'acide chlorhydrique concentrés et du substrat carboné dans le cas où celui-ci est un acide organique par exemple l'acide acétique. Une partie des ions $H^+$ nécessaires à la régulation du pH est amenée directement par la source de carbone et d'énergie présente dans le milieu d'alimentation dont le pH est alors acide, en général inférieur à 4.

La consigne de régulation du pH peut varier de 6 à 9, mais est préférentiellement fixée aux environs de la neutralité.

La température de dénitrification peut varier de 10 à 45°C, de préférence 20 à 40°C mais se situe encore préférentiellement aux environs de 30°C.

La biomasse est séparée de l'effluent dénitrifié qui peut être rejeté après avoir subi éventuellement d'autres traitements, tandis que la biomasse peut servir à ensemencer d'autres réacteurs.

L'invention va maintenant être illustrée au moyen des exemples de réalisation suivants donnés à titre illustratif et non limitatif.

**Exemples 1 à 7 :**

On réalise en anaérobiose, avec de l'acide acétique comme source de carbone une culture d'une population mixte sélectionnée conformément à l'invention, et dont la souche dominante est *Pseudomonas halodenitrificans.* On obtient une biomasse, très active du point de vue de la dénitrification que l'on utilise pour ensemencer un réacteur de type continu agité d'un volume utile de 0,45 litre. Le réacteur est alimenté par un milieu de dénitrification constitué par les effluents à traiter contenant des nitrates sous forme de $KNO_3$, $NaNO_3$ et NH4NO3 à une concentration de 500 mM auxquels sont ajoutées une source de carbone et d'énergie constituée par de l'acide acétique dans une quantité telle que le rapport de la concentration en acide acétique à celle en nitrates soit de 0,8 ainsi que 1 ml/l d'une solution d'oligo-éléments dont la composition est la suivante :

| Solution d'oligo-éléments pour 1 litre d'eau distillée | |
|---|---|
| $CuSO_4$, $5H_2O$ | 50 mg |
| $B(OH)_3$ | 100 mg |
| $MnSO_4$, $H_2O$ | 100 mg |
| $ZnSO_4$, $7H_2O$ | 100 mg |
| $Mo_7O_{24}(NH_4)_6$, $4H_2O$ | 1 g |
| $Co(NO_3)_2$, $6H_2O$ | 100 mg |

On a obtenu les résultats suivants rassemblés dans le tableau 1.

TABLEAU 1

| Exemple | $[(NO_3^-)]$ influent (mM) | Temps de séjour (j) | Productivité de dégradation des $NO_3^-$ | |
|---|---|---|---|---|
| | | | (mol/l.j) | (kg/m$^3$j) |
| 1 | 500 | 2,68 | 0,220 | 13,61 |
| 2 | 500 | 1,70 | 0,284 | 17,6 |
| 3 | 500 | 1,34 | 0,374 | 23,2 |
| 4 | 500 | 1,10 | 0,452 | 28,1 |
| 5 | 500 | 0,94 | 0,533 | 33,1 |
| 6 | 500 | 0,85 | 0,586 | 36,3 |
| 7 | 500 | 0,77 | 0,649 | 40,3 |

Pour ce qui concerne l'exemple 6, les données cinétiques suivantes ont également été calculées : la concentration en biomasse est de 4 g/l et la productivité spécifique de 9 kg de nitrate/kg de biomasse et par jour.

**Exemple 8** : On utilise un réacteur continu agité d'un volume utile de 1 litre alimenté par un effluent dont l'azote nitrique est constitué par $KNO_3$, $NaNO_3$, $NH_4NO_3$, et dont la concentration en nitrates est de 0,8 M.

A cet effet, est ajouté un substrat carboné constitué d'acide acétique de façon à ce que le rapport de la concentration en acide acétique à la concentration en nitrates soit de 0,8. La biomasse est la même que celle utilisée dans les exemples 1 à 7 et le temps de séjour est de 2,29 jours.

La productivité de la dégradation des nitrates est de 0,349 mole/l.j, soit 21,7 kg de nitrates/m$^3$.j.

**Exemple 9 :** Extrapolation de l'exemple 6 à un réacteur à membranes.

Pour une concentration en biomasse de 50 g/l, on obtient une productivité de 7,25 mole/l.j, soit 450 kg de $NO_3^-$/m$^3$.j.

Les résultats montrent que les performances obtenues dans le procédé de l'invention avec la mise en oeuvre d'un réacteur continu agité sont supérieures à toutes celles obtenues dans l'art antérieur pour le même type de réacteur.

## Revendications

1. Procédé de dénitrification biologique d'effluents renfermant de fortes concentrations en nitrates, par des bactéries, caractérisé en ce que la population mixte de bactéries dénitrifiantes mise en oeuvre est sélectionnée par repiquages successifs à partir de prélèvements des effluents à traiter et cultivée dans des milieux de culture comprenant les effluents à traiter eux-mêmes.

2. Procédé selon la revendication 1, caractérisé en ce que la suspension contenant la population mixte dénitrifiante sélectionnée est introduite dans un réacteur de dénitrification maintenu dans des conditions anaérobies alimenté par les effluents à traiter auxquels on ajoute un substrat carboné, une source de phosphore, des oligo-éléments, et en ce que le pH est maintenu à une valeur comprise entre 6 et 9.

3. Procédé selon la revendication 1, caractérisé en ce que le milieu de culture comprend outre les nitrates, un substrat carbone, une source de phosphore et des oligo-éléments.

4. Procédé selon la revendication 2 ou 3, caractérisé en ce que le substrat carboné est choisi parmi les acides organiques, les alcools, les effluents industriels chargés en polluants carbonés et leurs mélanges.

5. Procédé selon la revendication 2 ou 3, caractérisé en ce que la source de phosphore est constituée par des phosphates à une concentration supérieure à 16 mg de phosphore/g de biomasse.

6. Procédé selon la revendication 2 ou 3, caractérisé en ce que les oligo-éléments sont choisis parmi Mo, Fe, S, Cu, Mg, Mn et leurs mélanges.

7. Procédé selon la revendication 2 ou 3, caractérisé en ce que le contenu du réacteur et/ou le milieu de culture comprend en outre une source d'azote complémentaire différente des nitrates.

8. Procédé selon la revendication 2, caractérisé en ce que la biomasse est ensuite séparée de l'effluent dénitrifié.

9. Procédé selon la revendication 2, caractérisé en ce que le réacteur de dénitrification est choisi parmi les réacteurs discontinus « batch », les réacteurs continus agités, les réacteurs à lit fixe, les réacteurs à lit fluidisé, les réacteurs à lit agité, les réacteurs à membrane.

10. Procédé selon la revendication 2, caractérisé en ce que la pH est régulé grâce à la présence d'une pression partielle de $CO_2$ dans le réacteur.

11. Procédé selon la revendication 2, caractérisé en ce que le pH est régulé par addition d'un acide exogène dans le réacteur.

12. Procédé selon la revendication 2, caractérisé en ce que le pH est régulé par ajout simultané d'un acide exogène minéral fort et du substrat carboné si ce dernier est un acide organique.

13. Procédé selon la revendication 2, caractérisé en ce que la température de dénitrification est comprise entre 10 et 45°C.

14. Procédé de sélection d'une population mixte de bactéries performantes pour la dénitrification biologique d'effluents chargés en nitrates, caractérisé en ce que ladite population mixte de bactéries dénitrifiantes est sélectionnée par repiquages successifs à partir de prélèvements des effluents à traiter et cultivée dans des milieux de culture comprenant les effluents à traiter eux-mêmes.

15. Souche dénitrifiante Pseudomonas halodénitrificans déposée sous la référence CNCM I 1563 ou l'un de ses mutants.

**Patentansprüche**

1. Verfahren zur biologischen Denitrifikation von Abwässern, die hohe Konzentrationen an Nitraten enthalten, durch Bakterien, dadurch gekennzeichnet, daß die eingesetzte gemischte Population denitrifizierender Bakterien durch aufeinanderfolgende Verimpfungen aus Entnahmen zu behandelnder Abwässer selektiert wird und in Kulturmedien, welche die zu behandelnden Abwässer selbst enthalten, kultiviert wird.

2. Verfahren nach Anspruch 1, dadurch gekennzeichnet, daß die Suspension, welche die selektierte denitrifizierende gemischte Population enthält, in einen Denitrifikationsreaktor gebracht wird, der unter anaeroben Bedingungen gehalten wird und mit den zu behandelnden Abwässern gespeist wird, welchen man ein kohlenstoffhaltiges Substrat, eine Phosphorquelle, Spurenelemente zusetzt, und dadurch, daß der pH auf einem Wert zwischen 6 und 9 gehalten wird.

3. Verfahren nach Anspruch 1, dadurch gekennzeichnet, daß das Kulturmedium darüber hinaus die Nitrate, ein kohlenstoffhaltiges Substrat, eine Phosphorquelle, Spurenelemente enthält.

4. Verfahren nach Anspruch 2 oder 3, dadurch gekennzeichnet, daß das kohlenstoffhaltige Substrat unter den organischen Säuren, den Alkoholen, den mit kohlenstoffhaltigen Verunreinigungen beladenen industriellen Abwässern und ihren Mischungen gewählt wird.

5. Verfahren nach Anspruch 2 oder 3, dadurch gekennzeichnet, daß die Phosphorquelle aus Phosphaten in einer Konzentration größer als 16 mg Phosphor pro Gramm Biomasse besteht.

6. Verfahren nach Anspruch 2 oder 3, dadurch gekennzeichnet, daß die Spurenelemente unter Mo, Fe, S, Cu, Mg, Mn und ihren Mischungen gewählt werden.

7. Verfahren nach Anspruch 2 oder 3, dadurch gekennzeichnet, daß der Reaktorinhalt und/oder das Kulturmedium ferner eine zusätzliche, von den Nitraten verschiedene Stickstoffquelle umfaßt.

8. Verfahren nach Anspruch 2, dadurch gekennzeichnet, daß die Biomasse anschließend von dem denitrifizierten Abwasser getrennt wird.

9. Verfahren nach Anspruch 2, dadurch gekennzeichnet, daß der Denitrifikationsreaktor unter den diskontinuierlichen ("Batch-")Reaktoren, den gerührten kontinuierlichen Reaktoren, den Reaktoren mit festem Bett, den Reaktoren mit fluidisiertem Bett, den Reaktoren mit gerührtem Bett, den Reaktoren mit Membran gewählt wird.

10. Verfahren nach Anspruch 2, dadurch gekennzeichnet, daß der pH mit Hilfe des Vorhandenseins eines $CO_2$-Partialdrucks in dem Reaktor reguliert wird.

11. Verfahren nach Anspruch 2, dadurch gekennzeichnet, daß der pH durch Zugabe einer exogenen Säure in den Reaktor reguliert wird.

12. Verfahren nach Anspruch 2, dadurch gekennzeichnet, daß der pH durch gleichzeitige Zugabe einer starken exogenen Mineralsäure und des kohlenstoffhaltigen Substrats reguliert wird, wenn letzteres eine organische Säure ist.

13. Verfahren nach Anspruch 2, dadurch gekennzeichnet, daß die Temperatur der Denitrifikation zwischen 10 und 45°C liegt.

14. Verfahren zur Auswahl einer gemischten Population leistungsfähiger Bakterien für die biologische Denitrifikation mit Nitraten beladener Abwässer, dadurch gekennzeichnet, daß die genannte gemischte Population denitrifizierender Bakterien durch aufeinanderfolgende Verimpfungen aus Entnahmen zu behandelnder Abwässer selektiert wird und in Kulturmedien, welche die zu behandelnden Abwässer selbst enthalten, kultiviert wird.

15. Denitrifizierender Stamm Pseudomonas halodenitrificans, hinterlegt unter der Bezeichnung CNCM I 1563, oder einer seiner Mutanten.

## Claims

1. Process for the biological denitrification of effluents containing high nitrate concentrations, by bacteria, characterized in that the mixed population of denitrifying bacteria used is selected by successive seeding operations based on samples of the effluents to be treated and cultured in culture media incorporating the actual effluents to be treated.

2. Process according to claim 1, characterized in that the suspension containing the mixed, selected denitrifying population is introduced into a denitrification reactor maintained under anaerobic conditions supplied by the effluents to be treated and to which is added a carbon substrate, a phosphorus source, oligo-elements, and in that the pH is maintained at a value between 6 and 9.

3. Process according to claim 1, characterized in that the culture medium, apart from nitrates, comprises a carbon substrate, a phosphorus source and oligo-elements.

4. Process according to claim 2 or 3, characterized in that the carbon substrate is chosen from among organic acids, alcohols, industrial effluents containing carbon pollutants and mixtures thereof.

5. Process according to claim 2 or 3, characterized in that the phosphorus source is constituted by phosphates having a concentration higher than 16 mg of phosphorus/g of biomass.

6. Process according to claim 2 or 3, characterized in that the oligo-elements are chosen among Mo, Fe, S, Cu, Mg, Mn and mixtures thereof.

7. Process according to claim 2 or 3, characterized in that the content of the reactor and/or the culture medium also comprises a complimentary nitrogen source different from the nitrates.

8. Process according to claim 2, characterized in that the biomass is then separated from the denitrified effluent.

9. Process according to claim 2, characterized in that the denitrification reactor is chosen from among discontinuous, batch reactors, continuous stirred reactors, stationary bed reactors, fluidized bed reactors, stirred bed reactors and membrane reactors.

10. Process according to claim 2, characterized in that the pH is adjusted through the presence of a partial $CO_2$ pressure in the reactor.

11. Process according to claim 2, characterized in that the pH is adjusted by adding an exogenous acid to the reactor.

12. Process according to claim 2, characterized in that the pH is adjusted by the simultaneous addition of a strong, mineral, exogenous acid and the carbon substrate, if the latter is an organic acid.

13. Process according to claim 2, characterized in that the denitrification temperature is between 10 and 45°C.

14. Process for the selection of a mixed population of high performance bacteria for the biological denitrification of nitrate-containing effluents, characterized in that said mixed population of denitrifying bacteria is selected by successive seeding operations based on samples of the effluents to be treated and cultured in culture media incorporating the actual effluents to be treated.

15. Denitrifying strain Pseudomonas halodénitrificans filed under reference CNCM I 1563 or one of its mutants.